# EUROPÄISCHE PATENTSCHRIFT

(11) **EP 0 495 174 B1**
(45) Veröffentlichungstag und Bekanntmachung des Hinweises auf die Patenterteilung: **21.09.1994**
(21) Anmeldenummer: 91119600.4
(22) Anmeldetag: 16.11.1991
(51) Int. Cl.: C07H 15/04

(54) **Verfahren zur Herstellung von Alkylglycosiden und Alkylpolyglycosiden**
Method for the preparation of alkylglycosides and alkylpolyglycosides
Procédé de préparation d'alkylglycosides et d'alkylpolyglycosides

(30) Priorität: 17.01.1991 DE 4101252
(43) Veröffentlichungstag der Anmeldung: 22.07.1992
(73) Patentinhaber: HÜLS AKTIENGESELLSCHAFT, 45764 Marl (DE)
(72) Erfinder: Schmidt, Stefan, Dr., W-4350 Recklinghausen (DE)

(56) Entgegenhaltungen:
- EP-A- 0 077 167
- EP-A- 0 132 046
- EP-A- 0 252 250

## Beschreibung

Die Erfindung betrifft ein einstufiges Verfahren zur Herstellung von Alkylglycosiden und Alkylpolyglycosiden durch säurekatalysierte Umsetzung von Sacchariden mit Alkoholen mit 12 bis 20 C-Atomen.

Alkylglycoside und Alkylpolyglycoside mit C₁₂- bis C₂₀-Alkylresten können ganz oder teilweise aus nachwachsenden Rohstoffen hergestellt werden. Diese Alkylglycoside und Alkylpolyglycoside gewinnen wegen ihrer interessanten Tensideigenschaften bei gleichzeitig sehr guter biologischer Abbaubarkeit zunehmend an Bedeutung. Für Anwendungen im Haushalt und im Kosmetikbereich müssen diese Produkte hohen ästhetischen Ansprüchen genügen. Man ist daher an Verfahren interessiert, nach denen Alkylglycoside und Alkylpolyglycoside in transparenten, farblich schönen wäßrigen Lösungen hergestellt werden können.

Zur Herstellung von Alkylglycosiden und Alkylpolyglycosiden mit langkettigen Alkylgruppen kann man zunächst durch Glycosidierung von Sacchariden mit kurzkettigen Alkoholen Alkylglycoside und Alkylpolyglycoside mit C₁- bis C₆-Alkylgruppen herstellen. Diese Produkte werden dann mit langkettigen Alkoholen durch Umglycosidierung bei erhöhter Temperatur in die gewünschten Alkylglycoside und Alkylpolyglycoside übergeführt. Die so hergestellten Produkte sind jedoch dunkel gefärbt.

Nach EP 0 165 721 kann die Farbe derartiger Produkte durch mehrstufige Bleichung mit Wasserstoffperoxid verbessert und durch Zusatz von Schwefeldioxid freisetzenden Verbindungen stabilisiert werden. Der Aufhellungseffekt ist ohne Schwefeldioxid von nur kurzer Dauer.

In EP 0 077 167 wird ein einstufiges Herstellverfahren beschrieben, bei dem eine Aldose oder eine Ketose direkt mit einem langkettigen Alkohol im Molverhältnis von 1 : 1,25 bis 1 : 4 umgesetzt wird. Die Reaktion wird bei geringen Wassergehalten in Gegenwart eines Reduktionsmittels durchgeführt. Als Reduktionsmittel dient überwiegend hypophosphorige Säure. Das ebenfalls eingesetzte Natriumhypophosphit wird in Mengen von unter 0,4 %, bezogen auf das Saccharid, angewandt. Nach der Reaktion wird mit Alkali neutralisiert, worauf überschüssiger langkettiger Alkohol abdestilliert wird.

Wir haben gefunden, daß dieses Verfahren bei Einsatz von Alkoholen mit 12 bis 20 C-Atomen zu deutlich gefärbten Produkten führt.

Aufgabe der vorliegenden Erfindung war es deshalb, bei der Herstellung von Alkylglycosiden und Alkylpolyglycosiden in einer Stufe aus Sacchariden und Alkoholen mit 12 bis 20 C-Atomen in Gegenwart von Natriumhypophosphit und von bis zu 1,3 % Wasser die Farbe der Produkte zu verbessern.

Die Aufgabe wird erfindungsgemäß dadurch gelöst, daß man ein Saccharid/Alkohol-Molverhältnis von 1 : 5 bis 1 : 10 einstellt und nach der Reaktion mit Alkalihydroxid, gelöst in einem Alkohol mit 1 bis 4 C-Atomen, neutralisiert.

Als Saccharide können Aldosen und Ketosen eingesetzt werden. Beispiele dafür sind Glucose, Mannose, Galaktose und Fructose. Dabei wird Glucose vorzugsweise verwendet.

Für das vorliegende Verfahren geeignete Alkohole sind beispielsweise Laurylalkohol, Myristyl-, Palmityl- und Stearylalkohol. Es können auch Gemische von Alkoholen eingesetzt werden. Vorzugsweise verwendet man Alkohole mit 12 bis 16 C-Atomen.

Das Saccharid/Alkohol-Molverhältnis liegt bevorzugt im Bereich von 1 : 5 bis 1 : 8.

Als Katalysatoren sind Mineralsäuren und starke organische Säuren geeignet. Beispiele dafür sind Schwefelsäure, Phosphorsäure und p-Toluolsulfonsäure. Der Katalysator wird vorzugsweise in Konzentrationen von 0,2 bis 5 %, bezogen auf das Saccharid, eingesetzt.

Die Reaktion wird meist bei einer Temperatur von 80 bis 140 °C durchgeführt. Dabei werden Temperaturen von 90 bis 120 °C besonders bevorzugt.

Man führt die Reaktion in Gegenwart von 0,5 bis 5 % Natriumhypophosphit, bezogen auf das eingesetzte Saccharid, durch. Dabei werden Mengen von 1 bis 2 % bevorzugt.

Es muß darauf geachtet werden, daß der Wassergehalt, der vor der Reaktion berechnet und während der Reaktion durch Karl-Fischer-Titration bestimmt werden kann, 1,3 %, bezogen auf die Summe aus Saccharid und langkettigem Alkohol, nicht übersteigt. Bei höheren Wassergehalten wird die Färbung der Produkte deutlich dunkler.

Während der Reaktion wird gebildetes Wasser sofort abdestilliert. Dadurch kann der Wasseranteil auf einem niedrigen Niveau gehalten werden. Der Wassergehalt liegt vorzugsweise im Bereich von 0,1 bis 1 %.

Während der Reaktion werden Alkylglycoside und Alkylpolyglycoside hergestellt. Dabei werden alle Produkte, deren mittlerer Polymerisationsgrad größer als 1 ist, als Alkylpolyglycoside bezeichnet. Der mittlere Polymerisationsgrad liegt vorzugsweise im Bereich zwischen 1 und 8.

Man neutralisiert mit einer alkoholischen Alkalihydroxidlösung. Die hier als Lösemittel verwendeten kurzkettigen Alkohole sind beispielsweise Methanol, Ethanol, Isopropanol und Butanol. Vorzugsweise wird mit einer 1- bis 20%igen alkoholischen Alkalihydroxidlösung neutralisiert. Dabei wird methanolisches Kaliumhydroxid bevorzugt verwendet.

Nach dem erfindungsgemäßen Verfahren werden hellfarbige Alkylglycoside und Alkylpolyglycoside hergestellt. 50%ige wäßrige Lösungen dieser Produkte wiesen im allgemeinen Jodfarbzahlen von < 10 auf. Eine Nachbleichung, beispielsweise mit Wasserstoffperoxid, ist nicht erforderlich. Die Produkte können ohne weitere Behandlung direkt im Kosmetik- und im Waschmittelbereich eingesetzt werden.

Die folgenden Beispiele sollen die Erfindung verdeutlichen.

### Beispiel 1

225 g (1,25 Mol) wasserfreie Glucose, 1 215 g (6,25 Mol) eines 75 : 25-Gemisches aus n-Dodecanol und n-Tetradecanol, 3,6 g (0,035 Mol) konzentrierte Schwefelsäure und 3,8 g (0,035 Mol) Natriumhypophosphit werden unter Rühren im Wasserstrahlvakuum (20 hPa) 4 Stunden auf 100 °C erhitzt. Nach dieser Zeit ist im Reaktionsgemisch keine Glucose mehr nachzuweisen.

Wassergehalt zu Beginn der Reaktion: 0,30 %
Das Reaktionsgemisch wird auf ca. 50 °C abgekühlt und mit einer 2%igen methanolischen Kaliumhydroxid-Lösung auf pH 6 bis 7 eingestellt. Methanol und überschüssiger Fettalkohol werden am Rotationsverdampfer abgezogen. Der feste Rückstand wird im Verhältnis 1 : 1 mit Wasser abgemischt. Die so erhaltene Alkylpolyglycosid-Lösung ist wasserklar und vollständig farblos.
Jodfarbzahl: < 2

### Beispiel 2

225 g (1,25 Mol) wasserfreie Glucose, 1 215 g (6,25 Mol) eines 75 : 25-Gemisches aus n-Dodecanol und n-Tetradecanol, 4,6 g (0,045 Mol) konzentrierte Schwefelsäure und 3,7 g (0,034 Mol) Natriumhypophosphit werden unter Rühren im Wasserstrahlvakuum (20 hPa) 4 Stunden auf 110 °C erhitzt. Nach dieser Zeit ist im Reaktionsgemisch keine Glucose mehr nachzuweisen.

Wassergehalt zu Beginn der Reaktion: 0,30 %
Die Aufarbeitung erfolgt wie in Beispiel 1.
Jodfarbzahl: 4 bis 7

### Vergleichsbeispiel A

45 g (0,25 Mol) wasserfreie Glucose, 121 g (0,625 Mol) eines 75 : 25-Gemisches aus n-Dodecanol und n-Tetradecanol, 175 mg (1,75 mMol) konzentrierte Schwefelsäure und 430 mg (3,25 mMol) 50%ige hypophosphorige Säure werden unter Rühren im Wasserstrahlvakuum (20 hPa) 6 Stunden auf 100 °C erhitzt. Nach dieser Zeit ist im Reaktionsgemisch keine Glucose mehr nachzuweisen. Es treten jedoch schwarze Stippen auf.

Wassergehalt zu Beginn der Reaktion: 0,40 %
Die Aufarbeitung erfolgt wie in Beispiel 1. Zur Neutralisation wird jedoch festes, pulverisiertes Natriumcarbonat verwendet. Man erhält hier eine Alkylpolyglucosid-Lösung, die mit schwarzen Stippen durchsetzt ist.

### Vergleichsbeispiel B

45 g (0,25 Mol) wasserfreie Glucose, 121 g (0,625 Mol) eines 75 : 25-Gemisches aus n-Dodecanol und n-Tetradecanol, 500 mg (5 mMol) konzentrierte Schwefelsäure und 185 mg (1,75 mMol) Natriumhypophosphit werden unter Rühren im Wasserstrahlvakuum (20 hPa) auf 100 °C erhitzt. Nach einer Reaktionszeit von 6 Stunden ist im Ansatz noch deutlich Glucose nachweisbar.

Wassergehalt zu Beginn der Reaktion: 0,28 %
Die Aufarbeitung erfolgt wie in Vergleichsbeispiel A.
Jodfarbzahl: 700 bis 900

## Patentansprüche

1. Verfahren zur Herstellung von Alkylglycosiden und Alkylpolyglycosiden in einer Stufe durch säurekatalysierte Umsetzung von Sacchariden und Alkoholen mit 12 bis 20 C-Atomen in Gegenwart von Natriumhypophosphit und von bis zu 1,3 % Wasser, bezogen auf die Summe aus Sacchariden und Alkoholen,
dadurch gekennzeichnet,
daß das Saccharid/Alkohol-Molverhältnis bei 1 : 5 bis 1 : 10 liegt und nach beendeter Reaktion mit Alkalihydroxid, gelöst in einem Alkohol mit 1 bis 4 C-Atomen, neutralisiert wird.

2. Verfahren nach Anspruch 1,
dadurch gekennzeichnet,
daß das Saccharid/Alkohol-Molverhältnis bei 1 : 5 bis 1 : 8 liegt.

3. Verfahren nach Anspruch 1,
dadurch gekennzeichnet,
daß mit einer 1- bis 20%igen Alkalihydroxidlösung neutralisiert wird.

4. Verfahren nach Anspruch 1,
dadurch gekennzeichnet,
daß man mit methanolischem Kaliumhydroxid neutralisiert.

5. Verfahren nach Anspruch 1,
dadurch gekennzeichnet,
daß man als Saccharid Glucose einsetzt.

6. Verfahren nach Anspruch 1,
dadurch gekennzeichnet,
daß für die Reaktion Alkohole mit 12 bis 16 C-Atomen verwendet werden.

7. Verfahren nach Anspruch 1,
dadurch gekennzeichnet,
daß die Reaktion in Gegenwart von 0,1 bis 1,0 % Wasser durchgeführt wird.

8. Verfahren nach Anspruch 1,
dadurch gekennzeichnet,
daß die Reaktion bei einer Temperatur von 80 bis 140 °C durchgeführt wird.

## Claims

1. A process for preparing alkylglycosides and alkylpolyglycosides in one step by the acid-catalysed reaction of saccharides and alcohols having 12 to 20 C atoms in the presence of sodium hypophosphite and of up to 1.3% water, based on the sum of saccharides and alcohols, characterized in that the saccharide/alcohol molar ratio is from 1:5 to 1:10 and, once the reaction is complete, neutralization is carried out using alkali metal hydroxide, dissolved in an alcohol having from 1 to 4 C atoms.

2. A process according to claim 1, characterized in that the saccharide/alcohol molar ratio is from 1:5 to 1:8.

3. A process according to claim 1, characterized in that neutralization is carried out using an alkali metal hydroxide solution which is of from 1 to 20% strength.

4. A process according to claim 1, characterized in that neutralization is carried out using methanolic potassium hydroxide.

5. A process according to claim 1, characterized in that glucose is employed as the saccharide.

6. A process according to claim 1, characterized in that alcohols having from 12 to 16 C atoms are used for the reaction.

7. A process according to claim 1, characterized in that the reaction is carried out in the presence of from 0.1 to 1% water.

8. A process according to claim 1, characterized in that the reaction is carried out at a temperature of from 80 to 140°C.

## Revendications

1. Procédé de préparation en un stade d'alkyl-glycosides et d'alkyl-polyglycosides, par réaction, catalysée avec un acide, de saccharides et d'alcools comportant de 12 à 20 atomes de carbone, en présence d'hypophosphite de sodium et d'une quantité d'eau allant jusqu'à 1,3 %, relativement à la somme des saccharides et des alcools,
caractérisé en ce que le rapport molaire entre saccharide et alcool se situe à une valeur de 1 : 5 à 1 : 10 et que, lorsque la réaction est terminée, on neutralise avec un hydroxyde alcalin en solution dans un alcool comportant de un à 4 atomes de carbone.

2. Procédé selon la revendication 1,
caractérisé en ce que le rapport molaire entre saccharide et alcool se situe à une valeur de 1 : 5 à 1 : 8.

3. Procédé selon la revendication 1,
caractérisé en ce que l'on neutralise avec une solution renfermant de 1 à 20 % d'hydroxyde alcalin.

4. Procédé selon la revendication 1,
caractérisé en ce que l'on neutralise avec une solution méthanolique d'hydroxyde de potassium.

5. Procédé selon la revendication 1,
caractérisé en ce que l'on utilise le glucose comme saccharide.

6. Procédé selon la revendication 1,
caractérisé en ce qu'on utilise, pour la réaction, des alcools comportant de 12 à 16 atomes de carbone.

7. Procédé selon la revendication 1,
caractérisé en ce que l'on effectue la réaction en présence de 0,1 à 1,0 % d'eau.

8. Procédé selon la revendication 1,
caractérisé en ce que l'on effectue la réaction à une température de 80 à 140°C.
